# EUROPÄISCHE PATENTSCHRIFT

(11) **EP 1 381 689 B1**
(45) Veröffentlichungstag und Bekanntmachung des Hinweises auf die Patenterteilung: **20.08.2008**
(21) Anmeldenummer: 02737987.4
(22) Anmeldetag: 23.04.2002
(51) Int. Cl.: C12P 19/04, C12N 1/20

(54) **EXOPOLYSACCHARIDPRODUKTION**
EXOPOLYSACCHARIDE PRODUCTION
PRODUCTION D'EXOPOLYSACCHARIDE

(30) Priorität: 24.04.2001 DE 10120061
(43) Veröffentlichungstag der Anmeldung: 21.01.2004
(73) Patentinhaber: Prof. Dr. Lubitz, Werner, 3420 Klosterneuburg/Kritzendorf (AT)
(72) Erfinder: LUBITZ, Werner, A-3420 Klosterneuburg/Kritzendorf (AT); DENNER, Ewald, B., M., A-1020 Wien (AT)
(74) Vertreter: Dey, Michael
(86) Internationale Anmeldenummer: PCT/EP2002/004464
(87) Internationale Veröffentlichungsnummer: WO 2002/086099

(56) Entgegenhaltungen:
- EP-A- 0 507 234
- US-A- 5 854 034
- DENNER EWALD B M ET AL: "Sphingomonas pituitosa sp. nov., an exopolysaccharide-producing bacterium that secretes an unusual type of sphingan." INTERNATIONAL JOURNAL OF SYSTEMATIC AND EVOLUTIONARY MICROBIOLOGY, Bd. 51, Nr. 3, Mai 2001 (2001-05), Seiten 827-841, XP008016073 ISSN: 1466-5026
- LOBAS D ET AL: "Structure and physical properties of the extracellular polysaccharide PS-P4 produced by Sphingomonas paucimobilis P4 (DSM 6418)." CARBOHYDRATE RESEARCH. NETHERLANDS 3 JAN 1994, Bd. 251, 3. Januar 1994 (1994-01-03), Seiten 303-313, XP001148893 ISSN: 0008-6215
- POLLOCK T J: "GELLAN-RELATED POLYSACCHARIDES AND THE GENUS SPHINGOMONAS" JOURNAL OF GENERAL MICROBIOLOGY, SOCIETY FOR MICROBIOLOGY, READING, GB, Bd. 139, Nr. PART 8, 1. August 1993 (1993-08-01), Seiten 1939-1945, XP002035685 ISSN: 0022-1287
- ANSON A ET AL: "A BACTERIUM YIELDING A POLYSACCHARIDE WITH UNUSUAL PROPERTIES" JOURNAL OF APPLIED BACTERIOLOGY, OXFORD, GB, Bd. 62, Nr. 2, 1. Februar 1987 (1987-02-01), Seiten 147-150, XP000121053
- DATABASE EMBL [Online] 3. Juni 2000 (2000-06-03) MOORE E.R.B.: "Sphingomonas pituitosa partial 16S rRNA gene, strain EDIV" retrieved from EBI Database accession no. AJ243751 XP002238096

### SEQUENZPROTOKOLL

<110> Prof. Dr. Lubitz, Werner
<120> Exopolysaccharidproduktion
<130> 23786PDE_CG
<140> 10120061.7
   <141> 2001-04-24
<160> 1
<170> PatentIn Ver. 2.1
<210> 1
   <211> 1446
   <212> DNA
   <213> Sphingomonas pituitosa
<400> 1
   aacgaacgct ggcggcatgc ctaacacatg caagtcgaac gagatccttc ggggtctagt 60
   ggcgcacggg tgcgtaacgc gtgggaatct gccttggggt tcggaataac tccccgaaag 120
   gggtgctaat accggatgat gtcgaaagac caaagattta tcgccctgag atgagcccgc 180
   gtaggattag ctagttggtg tggtaaaggc gcaccaaggc gacgatcctt agctggtctg 240
   agaggatgat cagccacact gggactgaga cacggcccag actcctacgg gaggcagcag 300
   tggggaatat tggacaatgg gcgaaagcct gatccagcaa tgccgcgtga gtgatgaagg 360
   ccttagggtt gtaaagctct tttacccggg aagataatga ctgtaccggg agaataagcc 420
   ccggctaact ccgtgccagc agccgcggta atacggaggg ggctagcgtt gttcggaatt 480
   actgggcgta aagcgcacgt aggcggcttt gtaagtcaga ggtgaaagcc tggagctcaa 540
   ctccagaact gcctttgaga ctgcatcgct tgaatccagg agaggtgagt ggaattccga 600
   gtgtagaggt gaaattcgta gatattcgga agaacaccag tggcgaaggc ggctcactgg 660
   actggtattg acgctgaggt gcgaaagcgt ggggagcaaa caggattaga taccctggta 720
   gtccacgccg taaacgatga taactagctg tccgggcact tggtgcttgg gtggcgcagc 780
   taacgcatta agttatccgc ctggggagta cggccgcaag gttaaaactc aaaggaattg 840
   acgggggcct gcacaagcgg tggagcatgt ggtttaattc gaagcaacgc gcagaacctt 900
   accagcgttt gacatggtag gacgacttcc agagatggat ttcttccctt cggggaccta 960
   cacacaggtg ctgcatggct gtcgtcagct cgtgtcgtga gatgttgggt taagtcccgc 1020
   aacgagcgca accctcgact ttagttacca tcattaagtt gggtacttta aagtaaccgc 1080
   cggtgataag ccggaggaag gtggggatga cgtcaagtcc tcatggccct tacgcgctgg 1140
   gctacacacg tgctacaatg gcaagtacag tgggcagcaa tcccgcgagg gtgagctaat 1200
   ctccaaaact tgtctcagtt cggattgttc tctgcaactc gagagcatga aggcggaatc 1260
   gctagtaatc gcggatcagc atgccgcggt gaatacgttc ccaggccttg tacacaccgc 1320
   ccgtcacacc atgggagttg ggttcacccg aaggcgttgc gctaactcag caatgagagg 1380
   caggcgacca cggtgggctt agcgactggg gtgaagtcgt aacaaggtag ccgtagggga 1440
   acctgc                                                            1446

## Beschreibung

Die vorliegende Erfindung betrifft ein neues Epoxypolysacchariderzeugendes Bakterium, das aus diesem Bakterium gewonnene Exopolysaccharid (bezeichnet als PS-EDIV) sowie die Verwendung des Exopolysaccharids.

Seit der Beschreibung durch Yabuuchi et al. (1990) ist die Gattung Sphingomonas ein stetig wachsendes Taxon (Takeuchi et al., 1993, 1995; Nohynek et al., 1996; Zipper et al., 1996; Balkwill et al., 1997; Kämpfer et al., 1997; Denner et al., 1999). Lobas et al. (Carbohydrate Research, 251 (1994) 303-313) beschreiben Sphingomonas paucimobilis P4 (DSM 6418), welcher das Exopolysaccharid PS-P4 produziert.

Es besteht ein großes Interesse an Sphingomonaden, da sie breitgefächerte katabolische Fähigkeiten aufweisen und somit ein großes Potenzial für biotechnologische Anwendungen haben. Beispielsweise können Sphingomonaden bei der Abwasserbehandlung zum Abbau von xenobiotischen Verbindungen eingesetzt werden, als bakterielle Antagonisten für phytopathogene Pilze dienen und auch zur Erzeugung von industriell nutzbaren Exopolysacchariden eingesetzt werden (Pollock, 1993).

Es war deshalb eine Aufgabe der vorliegenden Erfindung, einen neuen Mikroorganismus der Gattung Sphingomonas bereitzustellen, der für biotechnologische Anwendungen oder/und zur Erzeugung von industriell einsetzbaren Produkten verwendet werden kann.

Diese Aufgabe wird erfindungsgemäß gelöst durch ein neues Exopolysaccharid-erzeugendes Bakterium, welches hierin als EDIV^{T} oder als Sphingomonas pituitosa sp. nov. bezeichnet wird und bei der DSMZ - Deutsche Sammlung von Mikroorganismen und Zellkulturen GmbH, Mascheroder Weg 1b, 38124 Braunschweig unter der Eingangsnummer DSM 13101 bzw. DSM 14559 sowie bei Collection de l'Institut Pasteur-CIP, B.P: 52, 25, rue de Dr. Roux, 75724 Paris Cedex, France unter der Eingangsnummer CIP 106154T hinterlegt worden ist. Der erfindungsgemäße, neue Bakterienstamm ist in der Lage, ein hochviskoses extrazelluläres Polysaccharid in einem Saccharose enthaltenden Mineralmedium zu erzeugen.

Der erfindungsgemäße Mikroorganismus kann beispielsweise in tryptischer Sojabrühe (Oxoid), welche 15 % Glycerin enthält, bei -70 °C gelagert werden. Beim erfindungsgemäßen Stamm EDIV^{T} handelt es sich um ein gram-negatives, Oxidase-negatives und Katalase-positives, aerobes, nichtsporenbildendes, bewegliches Stäbchenbakterium mit einem respiratorischen Metabolismus. Es erzeugt ein gelbes intrazelluläres Pigment (Carotenoide), welches nicht-fluoreszierend ist und nicht aus dem Organismus herausdiffundieren kann. Kolonien des Mikroorganismus sind gelb, zirkulär, niederkonvex und glatt. Die Oxidasereaktion ist negativ und die Katalasereaktion ist positiv. Für den β-Galactosidasetest wurden positive Reaktionen erhalten. Negative Reaktionen wurden für die Nitratreduktion, dem Ureasetest, die Produktion von Indol, und für Arginindihydrolase, Gelatineverflüssigung, H₂S-Produktion und Citratnutzung erhalten.

Die folgenden Verbindungen werden von dem neuen Organismus assimiliert: N-Acetyl-D-glucosamin, L-Arabinose, p-Arbutin, D-Cellobiose, D-Galactose, D-Glucose, D-Mannose, D-Maltose, α-D-Melibiose, Saccharose, Salicin, D-Trehalose, D-Xylose, Acetat, Fumarat, DL-3-Hydroxybutyrat, L-Malat, Pyruvat, L-Alanin und L-Prolin.

Die folgenden Verbindungen werden von dem erfindungsgemäßen Organismus nicht assimiliert: D-Fructose, Gluconat, L-Rhamnose, D-Ribose, Adonitol, i-Inositol, Maltitol, D-Mannitol, D-Sorbitol, Putrescin, Propionat, cis-Aconitat, trans-Aconitat, Adipat, 4-Aminobutyrat, Azelat, Citrat, Glutarat, Itaconat, DL-Lactat, Mesaconat, Oxoglutarat, Suberat, b-Alanin, L-Aspartat, L-Histidin, L-Leucin, L-Ornithin, L-Phenylalanin, L-Serin, L-Tryptophan, 3-Hydroxybenzoat, 4-Hydroxybenzoat und Phenylacetat.

Die folgenden Verbindungen werden vom erfindungsgemäßen Mikroorganismus hydrolysiert: Esculin, pNP-β-Galactopyranosid, pNP-b-Glucuronid, pNP-α-Glucopyranosid, pNP-β-Xylopyranosid, Bis-pNP-Phosphat, pNP-Phenylphosphat, pNP-Phosphorylcholin, 2-Deoxythymidin-5'-pNP-phosphat, L-Alanin-pNP und L-Glutamat-γ-3-carboxy-pNA.

L-Prolin-pNA wird durch den Organismus nicht hydrolysiert.

Als hauptsächliches respiratorisches Isoprenoidchinonsystem des neuen Organismus wurde Ubichinon Q-10 gefunden. Bei der Analyse des Polyamingehalts wurde sym-Homospermidin (68,4 µmol g⁻¹, Trockengewicht) als Hauptverbindung und Spermidin (3,3 µmol g⁻¹, Trockengewicht) und Spermin (1,0 µmol g⁻¹, Trockengewicht) als Nebenbestandteile bestimmt.

Als Hauptbestandteile der polaren Lipide wurden Phosphatidylethanolamin, Phosphatidyldiethanolamin, Phosphatidyldimethylethanolamin, Phosphatidylglycerin, Diphosphatidylglycerin und Sphingoglycolipid bestimmt.

Das zelluläre Fettsäureprofil des Mikroorganismus EDIV^{T} setzt sich wie folgt zusammen: cis 18:1 (58,6 %), 16:0 (20,9 %) und 2-OH 14:0 (10,0 %) als Hauptbestandteile der Fettsäuren sowie kleinere Mengen an cis 16:1 und cis 17:0 c. Weiterhin wurde festgestellt, dass der G + C-Gehalt der DNA des erfindungsgemäßen Organismus 64,5 mol-% beträgt, ein Wert, der innerhalb des für Mitglieder des Genus Sphingomonas festgestellten Bereichs liegt (Yabuuchi et al., 1990).

Die 16S rDNA-Sequenz des Stammes EDIV^{T} wurde bestimmt und ist als SEQ ID NO:1 dargestellt. Es wurde ein hohes Maß an Sequenzähnlichkeit zwischen EDIV^{T} und Sphinomonas trueperi (DSM 7225) mit einer Ähnlichkeit von 99,4 % beobachtet.

Eine Analyse der 16S rRNA-Gensequenz ergab, dass der Stamm EDIV^{T} innerhalb der α-4 Subklasse der Proteobacterien eingeordnet werden kann.

**Hierin beschrieben wird somit auch die ribosomale DNA des Mikroorganismus** Sphingomonas pituitosa sp. nov., umfassend (a) die in SEQ ID NO:1 gezeigte Nukleotidsequenz, (b) eine der in SEQ ID NO:1 gezeigten Nukleotidsequenz inneerhalb der Degeneration des genetischen Codes entsprechende Sequenz, oder (c) eine Sequenz, die mit den Sequenzen von (a) oder/und (b) unter stringenten Bedingungen hybridisiert.

**Beschrieben werden auch Sequenzen,** die mit der in SEQ ID NO:1 gezeigten Nukleotidsequenz oder einer dieser Sequenz innerhalb der Degeneration des genetische Codes entsprechenden Sequenz unter stringenten Bedingungen hybridisieren. Der Ausdruck "Hybridisierung unter stringenten Bedingungen" wird hierin wie bei Sambrook et al. (Molecular Cloning, A Laboratory Manual, Cold Spring Harbor Laboratory Press (1989), 1.101-1.104) verwendet. Bevorzugt liegt eine stringente Hybridisierung gemäß der vorliegenden Erfindung vor, wenn nach Waschen für 1 Stunde mit 1 x SSC und 0,1 % SDS bei 50 °C, bevorzugt bei 55 °C, mehr bevorzugt bei 62 °C und am meisten bevorzugt bei 68 °C und mehr bevorzugt für 1 Stunde mit 0,2 x SSC und 0,1 % SDS (Natriumdodecylsulfat) bei 50 °C, bevorzugt bei 55 °C, mehr bevorzugt bei 62 °C und am meisten bevorzugt bei 68 °C ein positives Hybridisierungssignal beobachtet wird.

Wenn man den erfindungsgemäßen Mikroorganismus auf einem Medium züchtet, welches Saccharose und insbesondere Saccharose als Hauptkohlenstoff- und Energiequelle enthält, werden große Mengen an extrazellulärem Polysaccharid gebildet. Dieses Polysaccharid wird insbesondere in Form eines extrazellulären Schleims erhalten. Flüssige Kulturen sowie Kolonien auf Agarmedien waren hochviskos. Die Erfindung umfasst deshalb auch ein Polysaccharid, welches unter Verwendung des neuen Exopolysaccharid-erzeugenden Bakteriums hergestellt werden kann, wobei das Polysaccharid ein Sphingan ist und mindestens Rhamnose- und Glucoseeinheiten enthält, nicht jedoch Glucuronsäure.

Eine Analyse des Polysaccharids ergab, dass es Rhamnose- und Glucoseeinheiten enthält. Glucuronsäure ist in dem von EDIV^{T} erzeugten Polysaccharid nicht enthalten.

Das erfindungsgemäße Polysaccharid bzw. Exopolysaccharid kann selbstverständlich auch auf synthetische Weise hergestellt werden.

Das erfindungsgemäße Polysaccharid weist weiterhin ein Molekulargewicht von > 2500 kDa, mehr bevorzugt mindestens 2900 kDa und bis zu 3500 kDa, insbesondere bis zu 3100 kDa auf. Das Molekulargewicht beträgt besonders bevorzugt etwa 3000 kDa. Das erfindungsgemäße Polysaccharid weist zahlreiche vorteilhafte Eigenschaften auf, die es für viele Anwendungen interessant machen. So ist es pseudoplastisch und tixotroph, ist beständig gegenüber einem Kochen für 30 Minuten und ist extrem stabil bei Temperaturen zwischen 25 und 99 °C und innerhalb weiter pH-Bereiche, beispielsweise von pH 1 bis pH 12,3. Weiterhin kann es nach einer Autoklavbehandlung vollständig abgebaut werden. Die Bildung erfolgt bevorzugt unter Verwendung von logarithmisch wachsenden Zellen von EDIV^{T}, wobei diese Zellen bevorzugt für 48 Stunden bei 28 °C gezüchtet werden. Um eine hohe Viskosität zu erhalten, erfolgt das Wachstum der Kultur bevorzugt bei hoher Perturbation oder/und einer hohen Sauerstoffkonzentration.

Das erfindungsgemäße Polysaccharid kann den Sphinganen zugeordnet werden und eignet sich insbesondere zur Anwendung in der Lebensmitteltechnologie, z.B. als Gelbildner oder/und in der pharmazeutischen Technologie, z.B. zur Formulierung oder Einkapselung von Arzneimitteln. Insbesondere kann eine verzögerte Wirkstofffreigabe durch Beschichten bzw. Einkapseln von Wirkstoffen mit dem erfindungsgemäßen Polysaccharid erhalten werden.

Das erfindungsgemäße Polysaccharid kann aber auch vorteilhaft in der chemischen Industrie oder/und in der Medizin eingesetzt werden. Zu den vielfältigen Anwendungsmöglichkeiten zählen beispielsweise seine Verwendung als Hilfsstoff für Tissue Engineering, als Wundgel oder als Träger für Arzneimittel. Es eignet sich aber auch hervorragend als Träger für Farben und Lacke, als Träger von Klebstoffen oder als Biopolymer für abbaubare Kunststoffe. Weiterhin kann das erfindungsgemäße Polysaccharid als Bestandteil von Nahrungsmitteln, insbesondere Functional Food oder Novel Food Produkten eingesetzt werden.

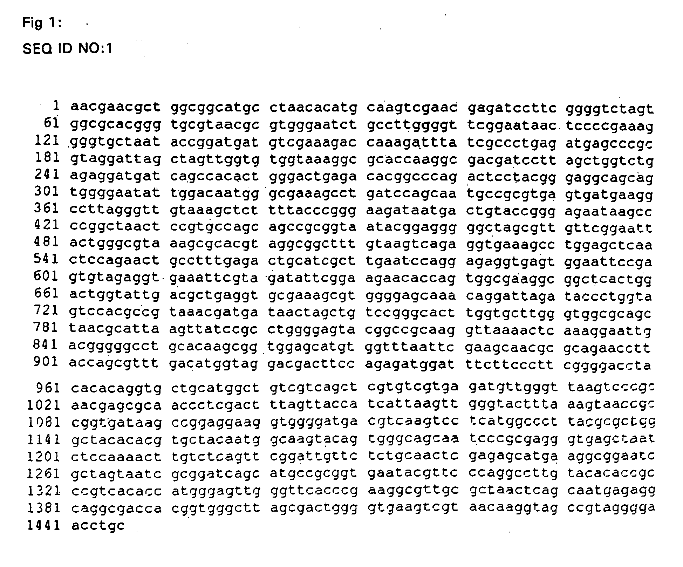

Figur 1 zeigt SEQ ID NO:1, die Sequenz der 16S ribosomalen RNA von Sphingomonas pituitosa sp. nov.

Die Erfindung wird durch die folgenden Beispiele weiter erläutert.

### Beispiel 1

Herstellung, Ausbeute und Charakterisierung des Exopolysaccharids (PS-EDIV)-Wachstumsbedingungen und Wachstumsmedium.

Für Wachstums- und Exopolysacchariduntersuchungen wurden Zellen von EDIV^{T} in einem Mineralsalzmedium gezüchtet, welches Saccharosemit einer Konzentration von 3, 6, 9, 12, 15, 18, 20 oder 22 % (Gew/Vol) oder Glucose bei Konzentrationen von 12 oder 15 % (Gew/Vol) enthielt. 100 ml des folgenden Mediums wurden mit 100 µl einer Zellsuspension (in 0,85 % NaCl-Lösung) inokuliert und in einem Wasserbadschüttler bei 28 °C für bis zu 8 Tage inkubiert. Die Rotationsfrequenz wurde hochreguliert (von 500 bis 800 U/min), wenn das Medium aufgrund der Exopolysacchariderzeugung zu viskos wurde. Medium: pH 7,3 (± 0,2), 1 g/l Dikaliumhydrogenphosphat, 0,5 g/l KCI, 0,01 g/l Fe(III)SO₄, 0,5 g/l MgSO₄·7H₂O, 3 g/l NaNO₃, Saccharose gemäß der gewünschten, oben angegebenen Konzentration bzw. Glucose gemäß der gewünschten, oben angegebenen Konzentration. Zur weiteren Extraktion des EPS wurde beispielsweise Saccharose bei einer Konzentration von 15 % (Gew/Vol) zugegeben. EDIV^{T} wurden bei 28 °C inkubiert.

Ein modifiziertes S-Medium, wie zuvor beschrieben (Fialho et al., 1991) ohne K₂SO₄ wurde ebenfalls verwendet, um die Ausbeute der EPS-Produktion nach einer Inkubation bei 28 °C für 144 Stunden abzuschätzen.

### Extraktion, Deacetylierung und Hydrolyse des Exopolysaccharids

Das Exopolysaccharid wurde mit dem dreifachen Volumen an 1-Propanol präzipitiert, wie von Azeredo und Olivera (1996) beschrieben. Die hochviskose Bakteriennäherlösung wurde dazu zunächst verdünnt (1:10) und bei 9000 U/min für 30 Minuten zentrifugiert, 3 mal mit Propanol gewaschen und lyophilisiert. Die Deacylierung wurde wie von Kang et al. (1982) beschrieben, durchgeführt. Das deacylierte Exopolysaccharid (EPS) wurde mit einem doppelten Volumen 1-Propanol präzipitiert und durch Lyophilisierung getrocknet. Zur Analyse der Zusammensetzung wurde sowohl das native als auch das deacylierte PS-EDIV mit Trifluoressigsäure, wie von Hashimoto und Murata, 1998 beschrieben, hydrolysiert. Die Trifluoressigsäure wurde unter Vakuum bei Raumtemperatur vollständig evaporiert. Das verbleibende, hydrolysierte PS-EDIV wurde in HPLC-Wasser (Merck) aufgenommen. Die PS-EDIV-Proben konnten gefroren bei -20 °C gelagert werden.

Die Monosaccharide des hydrolysierten PS-EDIV wurden durch Dünnschichtchromatograhie auf Silikagel-Platten (Kieselgel F₂₅₄, Merck) unter Verwendung von Aceton/Butanol/Wasser (40:5:5, vol/vol) und Butanol/Essigsäure/Wasser (4:6:1, vol/vol) als Lösungsmittelsysteme aufgetrennt. 1 bis 2 µl des PS-EDIV-Hydrolysats wurden auf die Dünnschichtplatte aufgebracht. Für Sphingane typische Komponenten, wie etwa Glucose, Glucuronsäure, Rhamnose und Mannose wurden als Referenzen verwendet. Die Bestandteile wurden durch Erwärmen der Dünnschichtplatten bei 110 °C für 5 Minuten nach Besprühen mit 10 % (vol/vol) Schwefelsäure in Ethanol (Hasimoto und Murata, 1998) bzw. α-Naphtol nachgewiesen.

Züsätzlich wurden Hydrolysate des PS-EDIV und Referenzsubstanzen (Glucose, Rhamnose, Mannose und Glucuronsäure) durch HPLC unter Verwendung einer Aminex HPX98-H-Säule mit 0,05 N H₂SO₄ als Eluant bei einer Betriebstemperatur von 60 °C und einer Strömungsgeschwindigkeit von 0,5 ml/min aufgetrennt.

Dabei wurde festgestellt, dass das von dem Stamm EDIV^{T} isolierte Polysaccharid Rhamnose und Glucose, nicht aber Glucuronsäure enthält.

### Literaturstellen

Auling., G,.. Busse, H.,J., Pilz, F., Webb., L., Kneifel. H. & Claus, D. (1991). Int. J. Syst. Bacteriol 41: 223-228.
Azeredo, J. & Olivera, R. (1996). Biotechnology Techniques 10: 341-344.
Balkwill, D. L., Drake, G. R., Reeves, R. H., Fredrikson, J. K., White, D. C., Ringelberg, D. B., Chandler, D. P. Romine, M. F., Kennedy, D. W. & Spandoni, C. M. (1997), Int. J. Syst. Bacteriol 47: 191-201.
Busse, J. & Auling, G. (1988). Syst. Appl. Microbiol. 11: 1-8.
Busse, H.-J., Bunka., S., Hensel, A. & Lubitz, W. (1997). Int. J. Syst. Bacteriol 47: 698-708.
Crescenzi, V. (1995). Europe. Biotechnol. Prog. 11: 251-259.
Denner, E. B. M., Kämpfer, P., Busse, H.-J. & Moore, E. R. B. (1999). Int. J. Syst. Bacteriol 49: 1103-1109.
De Vos, P. & De Ley, J. (1983). Int. J. Syst. Bacteriol 33: 487-509.
De Vos, P., Van Landschoot, A., Segers, P., Tytgat, R., Gillis, M., Bauwens, M., Rossau, R., Goor, M., Pot, B., Kersters, K., Lizzaraga, P. & De Ley, J. (1989). Int. J. Syst. Bacteriol 39: 35-49.
Hamana, K. & Matsuzaki, S. (1991). Can. J. Microbiol 39: 304-310.
Hashimoto, W. & Murato, K. (1998). Biosci. Biotechnol. Biochem 62: 1068-1074.
Jenkins, C. L., Andrews, A. G., McQuade, T. J. & Starr, M. P. (1979). Curr. Microbiol 3: 1-4.
Jukes, T. H. & Cantor, C. R. (1969). In Mammalian Protein Metabolism, S. 21-132. Edited by Munro, H.N. New York, Academic Press.
Kang, K.S., Veeder, G.T., Nirrasoul, P.J., Kaneto, T., & Cottrell, I.W. (1982) Agar-like polysaccharide produced by a Pseudomonas species: production and properties. Appl. Environ. Microbiol 43, 1086-1091.
Kämpfer, P. & Altwegg, M. (1992). J. Appl. Bacteriol 72: 341-351.
Kämpfer, P., Bark, K., Busse., H.-J., Auling, G. & Dott, W. (1992). Syst. Appl. Microbiol 15: 309-419.
Kämpfer, P. & Kroppenstedt, R. M. (1996). Can. J. Microbiol 42: 989-1005.
Kämpfer, P., Steiof, M. & Dott, W. (1991). Microbial. Ecol 21: 227-251.
Kämpfer, P., Denner, E. B. M., Meyer, S., Moore, E. R. B. & Busse, H.-J. (1997). Int. J. Syst Bacteriol 47: 577-583.
Kroppenstedt, R. M. (1982). GIT Lab. Med. 5: 266-275.
Moore, E. R. B., Wittich, R.-M., Fortnagel, P. & Timmis. K. N. (1993). Lett. Appl. Microbiol 17: 115-118.
Nohynek, L., Suhonen, E., Nurmiaho-Lassila, E.-L., Hantula, J. & Salkinoja-Salonen, M. (1996). Syst. Appl. Microbiol. 18: 527-538.
Pollock, T. J. (1993). J. Gen. Microbiol. 139: 1939-1945.
Scherer, F. & Kneifel, H. (1983). J. Bacteriol. 154: 1315-1322.
Stackebrandt, E., Murray, R. G. E. & Trüper, H. G. (1988). Int. J. Syst. Bacteriol 38: 321-325.
Sutherland, I. W. (1982). Adv. Microb. Physiol. 33: 79-150.
Sutherland, I. W. (1990). Camb. Stud. Biotechnol. 9: 1-151.
Takeuchi, M., Kawai, F., Shimada, Y., & Yokota, A: (1993). Syst. Appl. Microbiol 16: 227-238.
Takeuchi, M., Sakane, T., Yanagi, M., Yamasato, K., Hamana, K. & Yokota, A. (1995). Int. J. Syst. Bacteriol 45: 334-314.
Takeuchi, M., Sawada, H., Oyaizu, H. & Yokota, A. (1994). Int. J. Syst. Bacteriol 44: 308-314.
Tindall, B. J. (1990). FEMS Microbiol Lett 66: 199-202.
Woese, C. R., Blanz, P. & Hahn, C. M. (1984). Syst. Appl. Microbiol 5: 179-195.
Woese, C. R. (1987). Microbiol. Rev 51: 221-271.
Yabuuchi, E., Yano, I., Oyaizu, H., Hashimoto, Y., Ezaki, T. & Yamamoto, H. (1990). Microbiol. Immunol. 34: 99-119.
Zipper, C., Nickel, K., Angst, W. & Kohler, H.-P. (1996). Appl. Environm. Microbiol. 62: 4318-4322.

## Patentansprüche

1. Mikroorganismus Sphingomonas pitultosa sp. nov. DSM 14559.

2. Verwendung des Mikroorganismus nach Anspruch 1 zur Herstellung eines Polysaccharids, welches ein Sphingan ist und welches mindestens Rhamnose- und Glucoseeinheiten enthält, nicht jedoch Glucuronsäure, ein Molekulargewicht von mindestens 2900 kDa und bis zu 3100 kDa aufweist, pseudoplastisch und tixotroph ist, beständig gegenüber einem Kochen für 30 Minuten ist und extrem stabil bei Temperaturen zwischen 25 und 99 °C und von pH 1 bis pH 12,3 ist.

3. Polysaccharid, erhältlich durch Kultivierung des Mikroorganismus nach Anspruch 1,
**dadurch gekennzeichnet,**
**dass** es ein Sphingan ist und mindestens Rhamnose- und Glucoseeinheiten enthält, nicht jedoch Glucuronsäure, ein Molekulargewicht von mindestens 2900 kDa und bis zu 3100 kDa aufweist, pseudoplastisch und tixotroph ist, beständig gegenüber einem Kochen für 30 Minuten ist und extrem stabil bei Temperaturen zwischen 25 und 99 °C und von pH 1 bis pH 12,3 ist.

4. Verwendung des Polysaccharids nach Anspruch 3 in der Lebensmitteltechnologie, chemischen Industrie oder zur Herstellung eines Mittels in der Medizin oder/und pharmazeutischen Technologie.

5. Verwendung nach Anspruch 4 als Gelbildner oder/und zur Herstellung von Kapseln, insbesondere Gelkapseln.

6. Verwendung nach einem der Ansprüche 4 oder 5 als Hilfsstoff für Tissue Engineering, zur Herstellung eines Wundgels, als Träger für Arzneimittel, als Träger für Farben und Lacke, als Träger von Klebstoffen, als Biopolymer für abbaubare Kunststoffe oder/und zur Herstellung von Bestandteilen von "Novel Food"-Produkten.

## Claims

1. Microorganism Sphingomonas pituitosa sp. nov. DSM 14559.

2. Use of the microorganism according to claim 1 to produce a polysaccharide which is a sphingan and which contains at least rhamnose and glucose units but not glucuronic acid, has a molecular weight of at least 2900 kDa and up to 3100 kDa, is pseudoplastic and thixotrophic, is resistant to boiling for 30 minutes and is extremely stable at temperatures between 25 and 99°C and from pH 1 to pH 12.3.

3. Polysaccharide obtainable by culturing the microorganism according to claim 1,
**characterized in that**
it is a sphingan and contains at least rhamnose and glucose units but not glucuronic acid, has a molecular weight of at least 2900 kDa and up to 3100 kDa, is pseudoplastic and thixotrophic, is resistant to boiling for 30 minutes and is extremely stable at temperatures between 25 and 99°C and from pH 1 to pH 12.3.

4. Use of the polysaccharide according to claim 3 in food technology, the chemical industry or for producing an agent in medicine or/and pharmaceutical technology.

5. Use according to claim 4 as a gel former or/and for producing capsules and in particular gel capsules.

6. Use according to one of the claims 4 or 5 as an auxiliary agent for tissue engineering, for producing a wound gel, as an excipient for pharmaceutical preparations, as a carrier for paints and lacquers, as a carrier for adhesives, as a biopolymer for degradable plastics or/and for producing components of "novel food" products.

## Revendications

1. Micro-organisme *Sphingomonas pituitosa* sp. nov. DSM 14559.

2. Utilisation du micro-organisme selon la revendication 1 pour la fabrication d'un polysaccharide, qui est un sphingane et qui contient au moins des unités de rhamnose et de glucose, mais pas d'acide glucuronique, présente un poids moléculaire d'au moins 2900 kDa et allant jusqu'à 3100 kDa, est pseudoplastique et thixotrope, résiste à une cuisson de 30 minutes et est extrêmement stable à des températures allant de 25 à 99 °C et à un pH allant de 1 à 12,3.

3. Polysaccharide, que l'on peut obtenir par culture d'un micro-organisme selon la revendication 1, **caractérisé en ce qu'**il s'agit d'un sphingane et qu'il contient au moins des unités de rhamnose et de glucose, mais pas d'acide glucuronique, présente un poids moléculaire d'au moins 2900 kDa et allant jusqu'à 3100 kDa, est pseudoplastique et thixotrope, résiste à une cuisson de 30 minutes et est extrêmement stable à des températures allant de 25 à 99 °C et à un pH allant de 1 à 12,3.

4. Utilisation d'un polysaccharide selon la revendication 3 en technologie alimentaire, dans l'industrie chimique ou pour la fabrication d'une composition en médecine et/ou en technologie pharmaceutique.

5. Utilisation selon la revendication 4 en tant qu'agent de gélification et/ou pour la fabrication de gélules, en particulier de gélules en gel.

6. Utilisation selon l'une quelconque des revendications 4 ou 5 comme adjuvant pour l'ingénierie tissulaire, pour la fabrication d'un gel cicatrisant, comme support pour des médicaments, comme support pour colorants et peintures, comme support pour adhésifs, comme biopolymère pour matières plastiques biodégradables et/ou pour la fabrication de composants de produits de type « Novel Food ».
